(19) Europäisches Patentamt
European Patent Office
Office européen des brevets

(11) **EP 3 331 982 B1**

(12) **EUROPÄISCHE PATENTSCHRIFT**

(45) Veröffentlichungstag und Bekanntmachung des Hinweises auf die Patenterteilung:
**05.10.2022 Patentblatt 2022/40**

(21) Anmeldenummer: **16751218.5**

(22) Anmeldetag: **26.07.2016**

(51) Internationale Patentklassifikation (IPC):
**C11D 17/00** (2006.01)     **A61L 9/05** (2006.01)
**A61L 2/18** (2006.01)      **E03D 9/02** (2006.01)
**E03D 9/03** (2006.01)

(52) Gemeinsame Patentklassifikation (CPC):
**E03D 9/032; A61L 9/05; C11D 17/0056;**
**E03D 9/02;** A61L 2/18; E03D 2009/024

(86) Internationale Anmeldenummer:
**PCT/EP2016/067779**

(87) Internationale Veröffentlichungsnummer:
**WO 2017/025319 (16.02.2017 Gazette 2017/07)**

(54) **WC-STEIN UND WC-KÖRBCHEN**

BALL AND WC CAGE

BLOC W.-C. ET SUPPORT DE BLOC W.-C.

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **07.08.2015 DE 102015215135**

(43) Veröffentlichungstag der Anmeldung:
**13.06.2018 Patentblatt 2018/24**

(73) Patentinhaber: **Henkel AG & Co. KGaA**
**40589 Düsseldorf (DE)**

(72) Erfinder:
• **REICHERT, Christian**
  **76356 Weingarten (Baden) (DE)**
• **TREBBE, Uwe**
  **40219 Düsseldorf (DE)**
• **HORN, Michael**
  **40470 Düsseldorf (DE)**
• **CAPPLEMAN, Robert Stephen**
  **47269 Duisburg (DE)**
• **VÖLKER, Michael**
  **41379 Brüggen (DE)**

(56) Entgegenhaltungen:
WO-A1-2010/018006     WO-A1-2010/018006
WO-A1-2012/062914     WO-A1-2012/062914
US-B1- 6 376 442       US-B1- 6 376 442

**Beschreibung**

Die vorliegende Erfindung liegt auf dem Gebiet der WC-Steine, und betrifft einen WC-Stein, der aus mindestens einer Masse und einem Kern besteht. Die Erfindung betrifft auch ein WC-Körbchen umfassend einen erfindungsgemäßen WC-Stein.

WC-Steine mit zwei Massen sind bekannt, zum Beispiel aus der EP0791047 B1. Dort wird ein WC-Stein beschrieben, indem eine Masse durch eine weitere Masse mindestens teilweise umschlossen ist. Da beide Massen einen gemeinsamen Wirkstoff in unterschiedlichen Konzentrationen aufweisen, gibt es beim Abspülen, und beim völligen Übergang zur inneren Masse, eine Änderung der Konzentration des Wirkstoffes. Allerdings ist diese Änderung abrupt, und eine kontinuierliche Änderung ist mit dieser Technik ohne einen großen Aufwand nicht möglich.

Aus der DE102008028138A1 sind beschichtete WC-Steine bekannt, die Zusammensetzungen des WC-Steins und dessen Beschichtung sind dabei getrennt. Bei einer ersten Verwendung, z.B. bei der ersten Spülung, wird kein Wirkstoff vom WC-Stein an das Spülwasser abgegeben, sondern von dessen Beschichtung. Damit können optische Eigenschaften des WC-Steins eingestellt werden. Weiterhin sieht WO2010018006 vor, die WC-Steine zu beschichten, um optische Eigenschaften ein zustellen.

Aus der WO2006070209 sind zylindrische WC-Steine bekannt, die einen Kern aufweisen. Diese werden hauptsächlich als *in-tank* Keinigungsblöcke verwendet, und sind dazu bestimmt dauerhaft in Wasser zu liegen.

In der WO2012062914 werden mehrschichtige WC-Blocks oder-Steine beschrieben, wobei die unterschiedlichen Schichten, unterschiedlichen Wirkungen aufweisen können.

US 6 376 442 beschreibt WC-Blöcke mit konstanter Intensität des Parfüms, welche Blöcke in Form eines Kern-mit-Mantel Coextrudats hergestellt werden.

Ein wesentliches Merkmal der vorbekannten Austuhrungstormen des Standes der Technik ist, dass der Übergang zwischen den Schichten abrupt ist, dies ist insbesondere so wenn der Übergang einen Verbrauchsindikator darstellen soll. Es wurde noch keine Lösung gefunden um eine kontinuierliche Änderung der Wirkstoffabgabe zu realisieren.

Dieses Problem wird durch einen WC-Stein nach Anspruch 1 behoben. Die vorliegende Erfindung betrifft einen WC-Stein, welcher aus mindestens einem Mantel und einen Kern besteht. Der Mantel umfasst eine erste Zusammensetzung und der Kern umfasst eine zweite Zusammensetzung. Vorzugsweise unterscheiden sich die erste und die zweite Zusammensetzung durch mindestens eines der folgenden Merkmale: unterschiedliche Wirkstoffe, unterschiedliche Konzentration eines gleichen Wirkstoffes oder eine unterschiedliche Viskosität. Der Mantel ummantelt den Kern ringförmig, und er umschließt die Kernmasse teilweise. Der Kern ragt, auf mindestens einer Seite des WC-Steins, als ein Pol bis zur Oberfläche des WC-Steins und ist dabei exponiert. Der Verlauf des Kerns im WC-Steir ist derart ausgebildet, dass sich die Oberfläche der exponierten Fläche beim gleichmäßigen Abtragen der Oberfläche des WC-Steins, verändert.

Dabei ist es möglich mit verschiedenster geometrischer Außenform des WC-Steins, von Anfang an, gleichzeitig Wirkstoffe aus dem Mantel und aus dem Kern an die Spülung abzugeben. Dabei kann das Abgabeverhältnis von Wirkstoffen aus dem Mantel und aus dem Kern gezielt auf die Anzahl von Spülung angepasst werden. Insbesondere kann das Abgabeverhältnis so angepasst sein, dass es sich in kontinuierlicher Weise mit der Anzahl der Spülungen verändern. Es wird bevorzugt, dass die Veränderung kontinuierlich ist.

Vorzugsweise ist für die Veränderung der Oberfläche der exponierten Fläche des Kerns und somit die Veränderung der jeweils in die Spülung abgegebenen Wirkstoffzusammensetzung, die Ausgangslage am ungelösten WC-Stein ausschlaggebend. In anderen Worten, die Ausgangslage ist, vorzugsweise, der ungelöste WC-Stein im unbenutzten Originalzustand. Im Sinne der Erfindung ist mit exponiert die freiliegende Oberfläche gemeint.

Äußere, z.B. vollflächige, Beschichtungen des WC-Steins, die z.B. zu einem Glanz des unbenutzten WC-Steins sorgen, können zusätzlich vorgesehen sein. Solche Schichten sind in der Regel sehr dünn, so dass diese bei den ersten Spülungen, vorzugsweise schon bei den ersten 3 Spülungen, besonders bevorzugt bereits bei der ersten Spülung aufgelöst werden. Demensprechend fängt die eigentlich Abgabe von Wirkstoffe an das Spülwasser erst nach Auflösung dieser äußeren Beschichtung an. Daher, wird für die vorliegende Erfindung, als Ausgangslage für den ungelösten WC-Stein, den WC-Stein, ohne diese äußere Beschichtung verstanden.

In einer Ausgestaltung der Erfindung wird vorgesehen, dass sich die Oberfläche der exponierten Fläche des Kerns beim gleichmäßigen Abtragen der Oberfläche des WC-Steins im Zuge der fortlaufenden Nutzung vergrößert.

In einer besonders bevorzugten Ausgestaltung der Erfindung wird vorgesehen, dass sich die Oberfläche der exponierten Fläche des Kerns beim gleichmäßigen Abtragen der Oberfläche des WC-Steins, verkleinert. Damit wird es beispielsweise ermöglicht die Abgabe eines Wirkstoffs, welcher aus dem Kern abgegeben werden soll, so zu verringern, dass ein Verbraucher die Verringerung als Verbrauchsindikator wahrnehmen kann.

Es wird bevorzugt, dass das Flächenverhältnis der exponierten Oberfläche des Kerns zur exponierten Oberfläche des Mantels, bei dem ungelösten WC-Stein zwischen 5% und 67% ist, besonders bevorzugt zwischen 10% und 38%.

Eine gesamte Überdeckung des WC-Steins im ungenutzten Originalzustand des WC-Steins durch den Kern (99%-100% Überdeckung), oder durch den Mantel sind auch möglich, aber weniger bevorzugt. Durch die gesamte Überdeckung kann nämlich, bei dem ungelösten WC-Stein, zunächst nur eine Zusammensetzung abgegeben werden.

Es wird bevorzugt, dass nach Teilverbrauch des WC-Steins das Flächenverhältnis der exponierten Oberfläche des Kerns zur exponierten Oberfläche des Mantels zwischen 1:3 bis 1:1 ist.

Der WC-Stein umfasst einen zweiten Pol, der auf der zum ersten Pol gegenüberliegende Seite des WC-Steins zur Oberfläche ragt und dabei exponiert ist.

Vorzugsweise ist mindestens ein, vorzugsweise der mittlere Kernabschnitt als ein zylindrisches Segment ausgebildet. Insbesondere für zweipolige WC-Steine kann der Kern mit einer zu den beiden Polen im wesentlichen symmetrischen Verteilung erzeugt werden, sofern der mittlere Abschnitt zylindrisch ist.

Der erste Pol und der zweite Pol sind durch einen Strang verbunden.

Dadurch ergibt sich ein besserer Zeitverlauf der Wirkstoffabgabe während des Verbrauches des WC-Steins.

Bei den WC-Steinen der Erfindung, wird es bevorzugt, dass der Querschnittsverlauf des Kerns zwischen erstem Pol und zweitem Pol eine Querschnittsverkleinerung aufweist.

Gemäß der Erfindung, ist vorgesehen, dass der Mantel den Kern ringförmig ummantelt. Damit kann der Kern durch die Ringmitte erstreckt und auf jeweils einer Seite als erster und zweiter Pol entfaltet ausgeführt sein.

In einer alternativen Ausführung der Erfindung weist der Querschnittsverlauf des Kernes zwischen erstem Pol und zweitem Pol eine Querschnittsvergrößerung auf.

Es wird bevorzugt, dass der WC-Stein sphärisch ist. Die Sphärizität ist vorzugsweise zwischen 0,8 und 1, d. h. im Wesentlichen kugelförmig, insbesondere bevorzugt zwischen 0,85 und 1, ganz besonders bevorzugt zwischen 0,9 und 1 auf. Eine besonders bevorzugte Ausführung der Erfindung, sieht ein WC-Körbchen vor, dass mindestens 2, bevorzugt mindestens 3 sphärische WC-Steine umfasst die in einer Reihe angeordnet sind, wobei es weiterhin bevorzugt ist, dass die Pole der WC-Steine in einer zufälligen Anordnung vorliegen. Zufällige Anordnung bedeutet, dass die in der WC-Steinanordnung umfassenden WC-Steine, mit unterschiedlichen Pol-Orientierungen vorliegen. So ein WC-Körbchen ist günstiger herzustellen da eine Vororientierung der WC-Steine nicht notwendig ist. Es wurde herausgefunden, dass mit erfindungsgemäßen sphärischen WC-Steinen, eine Vororientierung nicht notwendig ist um das gewünschte Abgabeverhältnis, mit der Anzahl der Spülungen, zu liefern. Selbstverständlich können die WC-Steine auch orientiert sein, falls eine bestimmte ästhetische Form gewünscht wird, insbesondere falls der Kern und der Mantel unterschiedliche Farben aufweisen.

Die Sphärizität $\Psi$ eines Körpers K ist das Verhältnis der Oberfläche des Körpers zur Oberfläche einer Kugel gleichen Volumens:

$$\Psi = \frac{\pi^{\frac{1}{3}}\left(6V_p\right)^{\frac{2}{3}}}{A_p},$$

wobei $V_p$ das Volumen des Körpers und $A_p$ seine Oberfläche bezeichnet Durch die nahezu ideale kugelförmige Ausbildung des WC-Steins wird ein gleichmäßiges Abspülen des WC-Reinigungsblocks in der Art bewirkt, dass der WC-Stein seine Kugelform auch während bzw. nach den Abspülvorgängen und einem entsprechendem Abtrag des WC-Reinigungsblocks im Wesentlichen beibehält.

Der Durchmesser des kugelförmigen WC-Steins beträgt vorzugsweise zwischen 1 mm und 10 cm, bevorzugt zwischen 5 mm und 5 cm, insbesondere bevorzugt zwischen 1 cm und 3 cm.

Noch ein weiterer Erfindungsgegenstand ist ein zugehöriges Verfahren zur Herstellung eines rotationssymmetrischen WC-Reinigungsblocks, umfassend die Schritte

a) Mischen der Inhaltsstoffe für die Kernzusammensetzung, und Mischen der Inhaltsstoffe für die Mantelzusammensetzung,
b) Co-Extrusion der mindestens zwei Gemische,
c) Schneiden des extrudierten Strangs in Portionsstücke einer definierten Masse,
d) Verformung zu rotationssymmetrischen Körpern, so dass der Kern auf mindestens einer Seite des WC-Steins, als einen Pol bis zur Oberfläche ragt und dabei exponiert ist.

Dabei erfolgt die Verformung d) vorzugsweise in einer Kugelrollmaschine oder einer Presse. Eine bevorzugte Kugelrollmaschine ist eine mit drei rotierende Formen oder Walzen, in denen der Strang reingelegt werden kann, und durch Verkleinerung des Abstandes zwischen den Walzen der Strang durch die Walzenform geschnitten und zu einer Kugel geformt wird. Dadurch, dass der Strangabschnitt in einen kugelförmigen Raum mit identischen Volumen gepresst wird, wird die gewünschte erfindungsgemäße Endgeometrie erreicht.

Die Schritte a) und b) können auch kombiniert werden, also Mischen der Inhaltsstoffe im Extruder. Die Verfahrensschritte laufen gegebenenfalls bei unterschiedlichen Temperaturen ab, so dass zwischen den Schritten noch Heiz- oder Kühlschritte zwischengeschaltet sein können. Diese liegen im Ermessen des Fachmanns.

In einer bevorzugten Ausführungsform wird ein weiterer Verfahrensschritt im Anschluss an einen der Schritte b) oder c) durchgeführt, bei dem der extrudierte Strang mit einem Schmiermittel versehen wird. Hierzu wird ein permanent mit dem Schmiermittel versetzter Schwamm in Form eines Laufrades derart über den extrudierten Strang geführt, dass die Oberfläche ganz oder teilweise, vorzugsweise zu 10 bis 40%, mit Schmiermittel beaufschlagt ist. Die Zugabe des Schmiermittels verbessert hierbei die nachfolgende Kugelformung. Geeignete Schmiermittel sind insbesondere Stoffe, die beispielsweise als Tenside oder Abspülregulatoren in erfindungsgemäßen Rezepturen eingesetzt werden. Besonders bevorzugt wird dabei ein Schmiermittel, ausgewählt aus der Gruppe umfassend Dipropylenglykol, Paraffine, nichtionische Tenside, Polyethylenglykole sowie Gemische derselben, eingesetzt, insbesondere Dipropylenglykol.

Das Verfahren zur Herstellung des WC-Körbchens umfasst die oben beschriebene Schritte a) bis d) und umfasst weiterhin:

e) Bereitstellung von Kunststoffhalterung, vorzugsweise durch Spritzgussverfahren;
f) Einlegen der WC-Steine in die Kunststoffhalterung;
g) Verschließen der Kunststoffhalterung.

Vorzugsweise, werden zwischen den Schritten d) und f) die WC-Steine in einen Container zwischengelagert. Der Container bildet eine Art Puffer, und dadurch kann die Herstellung der WC-Steine von der Herstellung der WC-Körbchen entkoppelt werden. Vorzugsweise kommen die WC-Steine, die in den Körbchen verwendet werden, aus mindestens zwei Containern, wobei die Container WC-Steine mit unterschiedlichen Zusammensetzungen gelagert haben. Somit kann ein WC-Körbchen hergestellt werden, welches Wirkstoffe aus unterschiedlichen WC-Steinen mit unterschiedlichen Zusammensetzungen abgeben kann.

Zusammensetzung

Die erste und die zweite Zusammensetzung unterscheiden sich vorzugsweise durch mindestens durch das beinhalten unterschiedlicher Wirkstoffe und/oder durch eine unterschiedliche Konzentration eines gleichen Wirkstoffes, wobei die betroffene Wirkstoffe vorzugsweise aus der folgende Liste gewählt sind: Parfüm, Tensid(e), Farbstoffe, Abspülregulatoren, Bleichmittel, Builder, Säure oder Base, Antimikrobielle Wirkstoffe, Polymere. Es wird besonders bevorzugt, dass die betroffene Wirkstoffe ausgewählt sind aus mindestens eins von: Parfüm, Tensid(e), Farbstoffe. Bei einem WC-Stein indem sich die erste und die zweite Zusammensetzungen durch eine unterschiedliche Konzentration eines gleichen Wirkstoffes unterscheiden, wird es bevorzugt dass der Kern eine höhere Konzentration dieses Wirkstoffes aufweist. Vorzugsweise ist die höhere Konzentration im Kern mindestens 0,5 Gew.-% größer als im Mantel. Weiterhin wird bevorzugt, dass der Wirkstoff im Kern mit einer höheren Konzentration von mindestens 1 Gew.-% bis 10 Gew.-% als die Konzentration im Mantel vorliegt. Die Gew.-% Angabe ist immer im Verhältnis zur gesamten Zusammensetzung von 100%. Eine höhere Konzentration von x Gew.-% bedeutet x Prozentpunkte mehr.

Das erfindungsgemäße WC-Körbchen mit WC-Stein kann zudem in einem Verfahren zum Reinigen und/oder Beduften und/oder Desinfizieren von Spültoiletten eingesetzt werden dergestalt, dass das mit dem WC-Stein befüllte WC-Körbchen in die WC-Schüssel eingehängt wird und beim Betätigen der Toilettenspülung gelöste Inhaltsstoffe des WC-Steins in das Spülwasser gelangen und dort ihre reinigende und/oder duftende und/oder desinfizierende Wirkung entfalten können. Noch ein weiterer Erfindungsgegenstand ist daher ein Verfahren zum Reinigen und/oder Beduften und/oder Desinfizieren von Spültoiletten unter Verwendung eines erfindungsgemäßen WC-Körbchens mit WC-Stein.

Stoffe, die auch als Inhaltsstoffe von kosmetischen Mitteln dienen, werden nachfolgend ggf. gemäß der *International Nomenclature Cosmetic Ingredient* (INCI)-Nomenklatur bezeichnet. Chemische Verbindungen tragen eine INCI-Bezeichnung in englischer Sprache, pflanzliche Inhaltsstoffe werden ausschließlich nach Linné in lateinischer Sprache aufgeführt, sogenannte Trivialnamen wie "Wasser", "Honig" oder "Meersalz" werden ebenfalls in lateinischer Sprache angegeben. Die INCI-Bezeichnungen sind dem International Cosmetic Ingredient Dictionary and Handbook - Seventh Edition (1997) zu entnehmen, das von The Cosmetic, Toiletry, and Fragrance Association (CTFA), 1101 17th Street, NW, Suite 300, Washington, DC 20036, USA, herausgegeben wird und mehr als 9.000 INCI-Bezeichnungen sowie Verweise auf mehr als 37.000 Handelsnamen und technische Bezeichnungen einschließlich der zugehörigen Distributoren aus über 31 Ländern enthält. Das *International Cosmetic Ingredient Dictionary and Handbook* ordnet den Inhaltsstoffen eine oder mehrere chemische Klassen (*Chemical Classes*), beispielsweise *Polymeric Ethers,* und eine oder mehrere Funktionen (*Functions*), beispielsweise *Surfactants - Cleansing Agents,* zu, die es wiederum näher erläutert und auf die nachfolgend ggf. ebenfalls Bezug genommen wird.

Die Angabe CAS bedeutet, dass es sich bei der nachfolgenden Zahlenfolge um eine Bezeichnung des *Chemical Abstracts Service* handelt.

Im Rahmen der vorliegenden Erfindung stehen Fettsäuren bzw. Fettalkohole bzw. deren Derivate - soweit nicht anders angegeben - stellvertretend für verzweigte oder unverzweigte Carbonsäuren bzw. Alkohole bzw. deren Derivate mit vorzugsweise 6 bis 22 Kohlenstoffatomen, insbesondere 8 bis 20 Kohlenstoffatomen, besonders bevorzugt 10 bis 18 Kohlenstoffatomen, äußerst bevorzugt 12 bis 16 Kohlenstoffatomen, beispielsweise 12 bis 14 Kohlenstoffatomen. Erstere sind insbesondere wegen ihrer pflanzlicher Basis als auf nachwachsenden Rohstoffen basierend aus ökologischen Gründen bevorzugt, ohne jedoch die erfindungsgemäße Lehre auf sie zu beschränken. Insbesondere sind auch die beispielsweise nach der Roelenschen Oxo-Synthese erhältlichen Oxo-Alkohole bzw. deren Derivate mit vorzugsweise 7 bis 19 Kohlenstoffatomen, insbesondere 9 bis 19 Kohlenstoffatomen, besonders bevorzugt 9 bis 17 Kohlenstoffatomen, äußerst bevorzugt 11 bis 15 Kohlenstoffatomen, beispielsweise 9 bis 11, 12 bis 15 oder 13 bis 15 Kohlenstoffatomen, entsprechend einsetzbar.

Parfüm

Die Zusammensetzungen enthalten jeweils einen oder mehrere Duftstoffe, vorzugsweise in einer Menge von 0,01 bis 15 Gew.-%, insbesondere 1 bis 11 Gew.-%, besonders bevorzugt 1 bis 8 Gew.-%. Vorzugsweise ist die Konzentration im Mantel zwischen 1 Gew.-% und 3 Gew.-% und die Konzentration im Kern mindestens 0,5 Gew.-% größer, besonders bevorzugt zwischen 1,5 Gew.-% und 8 Gew.-%.

Als eine Parfümkomponente kann dabei d-Limonen enthalten sein. In einer besonders bevorzugten Ausführungsform enthält der erfindungsgemäße WC-Reinigungsblock dabei ein Parfüm aus ätherischen Ölen (auch als essentielle Öle bezeichnet). Als solche sind beispielsweise Pine-, Citrus-, Jasmin-, Patchouly-, Rosen- oder Ylang-Ylang-Öl im Sinne dieser Erfindung einsetzbar. Ebenfalls geeignet sind Muskateller-Salbeiöl, Kamillenöl, Lavendelöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliol, Orangenschalenöl und Sandelholzöl.

Um wahrnehmbar zu sein, muss ein Riechstoff flüchtig sein, wobei neben der Natur der funktionellen Gruppen und der Struktur der chemischen Verbindung auch die Molmasse eine wichtige Rolle spielt. So besitzen die meisten Riechstoffe Molmassen bis etwa 200 Dalton, während Molmassen von 300 Dalton und darüber eher eine Ausnahme darstellen. Aufgrund der unterschiedlichen Flüchtigkeit von Riechstoffen verändert sich der Geruch eines aus mehreren Riechstoffen zusammengesetzten Parfüms während des Verdampfens, wobei man die Geruchseindrücke in "Kopfnote" (top note), "Herz- bzw. Mittelnote" (middle note bzw. body) sowie "Basisnote" (end note bzw. dry out) unterteilt.

Haftfeste Riechstoffe, die im Rahmen der vorliegenden Erfindung vorteilhafterweise in den Parfümölen einsetzbar sind, sind beispielsweise die ätherischen Öle wie Angelikawurzelöl, Anisöl, Arnikablütenöl, Basilikumöl, Bayöl, Champacablütenöl, Edeltannenöl, Edeltannenzapfenöl, Elemiöl, Eukalyptusöl, Fenchelöl, Fichtennadelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaivabalsamöl, Korianderöl, Krauseminzeöl, Kümmelöl, Kuminöl, Lemongrasöl, Moschuskörneröl, Myrrhenöl, Nelkenöl, Neroliöl, Niaouliöl, Olibanumöl, Origanumöl, Palmarosaöl, Patschuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Sternanisöl, Thujaöl, Thymianöl, Verbenaöl, Vetiveröl, Wacholderbeeröl, Wermutöl, Wintergrünöl, Ylang -Ylang-Öl, Ysop-Öl, Zimtöl, Zimtblätteröl sowie Zypressenöl.

Aber auch die höhersiedenden bzw. festen Riechstoffe natürlichen oder synthetischen Ursprungs können im Rahmen der vorliegenden Erfindung vorteilhafterweise als haftfeste Riechstoffe bzw. Riechstoffgemische in den Parfümölen eingesetzt werden. Zu diesen Verbindungen zählen die nachfolgend genannten Verbindungen sowie Mischungen aus diesen: Ambrettolid, $\alpha$-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylakohol, Borneol, Bornylacetat, $\alpha$-Bromstyrol, n-Decylaldehyd, n-Dodecyl-aldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Di-methylether, Hydroxyzimtaldehyd, Hydroxyzimtalkohol, Indol, Iron, Isoeugenol, Isoeugenolmethylether, Isosafrol, Jasmon, Kampfer, Karvakrol, Karvon, p- Kresolmethyl-ether, Cumarin, p-Methoxyacetophenon, Methyl-n-amylketon, Methylanthranilsäuremethylester, p-Methylacetophenon, Methylchavikol, p-Methylchinolin, Methyl-$\beta$-naphthylketon, Methyl-n-nonylacetaldehyd, Methyl-n-nonylketon, Muskon, $\beta$-Naphtholethylether, $\beta$-Naphthol-methylether, Nerol, Nitrobenzol, n-Nonylaldehyd, Nonylakohol, n-Octylaldehyd, p-Oxy-Acetophenon, Pentadekanolid, $\beta$-Phenylethylakohol, Phenylacetaldehyd-Dimethylacetal, Phenylessigsäure, Pulegon, Safrol, Salicylsäureisoamylester, Salicylsäuremethylester, Salicylsäurehexylester, Salicylsäurecyclohexylester, Santalol, Skatol, Terpineol, Thymen, Thymol, $\gamma$-Undelacton, Vanilin, Veratrumaldehyd, Zimtaldehyd, Zimtalkohol, Zimtsäure, Zimtsäureethylester, Zimtsäurebenzylester.

Zu den leichter flüchtigen Riechstoffen, die im Rahmen der vorliegenden Erfindung in den Parfümöl vorteilhaft einsetzbar sind, zählen insbesondere die niedriger siedenden Riechstoffe natürlichen oder synthetischen Ursprung, die allein oder in Mischungen eingesetzt werden können. Beispiele für leichter flüchtige Riechstoffe sind Alkyisothiocyanate (Alkylsenföle), Butandion, Limonen, Linalool, Linaylacetat und -propionat, Menthol, Menthon, Methyl-n-heptenon, Phellandren, Phenylacetaldehyd, Terpinylacetat, Citral, Citronellal.

Tenside

Der erfindungsgemäße WC-Stein enthält mindestens ein nichtionisches Tensid, wobei ein $C_{12-22}$ Fettalkoholakoxylat mit einem Ethoxylierungsgrad von 12 bis 28 enthalten ist, sowie mindestens ein Alkylbenzolsulfonat und mindestens ein Olefinsulfonat. Daneben können weitere Tenside enthalten sein.

Bei den Alkylbenzolsulfonaten sind dabei insbesondere solche mit etwa 12 C-Atomen im Alkylteil bevorzugt, etwa lineares Natrium-C10-13-Alkylbenzolsulfonat. Bevorzugte Olefinsulfonate weisen eine Kohlenstoffkettenlänge von 14 bis 16 auf. Der erfindungsgemäße WC-Reinigungsblock enthält dabei bevorzugt 10 bis 70 Gew.-%, vorzugsweise 20 bis 65 Gew.-%, besonders bevorzugt 20 bis 30 Gew.-% Alkylbenzolsulfonat und bevorzugt 10 bis 30 Gew.-%, vorzugsweise 15 bis 30 Gew.-%, besonders bevorzugt 15 bis 25 Gew.-% Olefinsulfonat.

Nichtionische Tenside

Zusätzlich zu dem $C_{12-22}$ Fettalkoholakoxylat mit einem Ethoxylierungsgrad von 12 bis 28 können weitere nichtionische Tenside enthalten sein. Weitere geeignete nichtionische Tenside im Rahmen der Erfindung können Alkoxylate sein wie Polyglycolether, Fettalkoholpolyglycolether, Alkylphenolpolyglycolether, endgruppenverschlossene Polyglycolether, Mischether und Hydroxymischether und Fettsäurepolyglycolester. Ebenfalls verwendbar sind Ethylenoxid/Propylenoxid-Blockpolymere, Fettsäurealkanolamide und Fettsäurepolyglycolether. Eine weitere wichtige Klasse nichtionischer Tenside, die erfindungsgemäß verwendet werden kann, sind die Polyol-Tenside und hier besonders die Glykotenside, wie Alkylpolyglykoside und Fettsäureglucamide. Besonders bevorzugt sind die Alkylpolyglykoside, insbesondere die Alkylpolyglucoside, sowie vor allem die Fettalkoholalkoxylate (Fettalkoholpolyglycolether).

Bevorzugte Fettalkoholalkoxylate sind mit Ethylenoxid (EO) und/oder Propylenoxid (PO) alkoxylierte, unverzweigte oder verzweigte, gesättigte oder ungesättigte $C_{8-22}$-Alkohole mit einem Alkoxylierungsgrad bis zu 30, vorzugsweise ethoxylierte $C_{12-22}$-Fettalkohole mit einem Ethoxylierungsgrad von weniger als 30, bevorzugt 12 bis 28, insbesondere 20 bis 28, besonders bevorzugt 25, beispielsweise $C_{16-18}$-Fettalkoholethoxylate mit 25 EO.

Alkylpolyglykoside sind Tenside, die durch die Reaktion von Zuckern und Alkoholen nach den einschlägigen Verfahren der präparativen organischen Chemie erhalten werden können, wobei es je nach Art der Herstellung zu einem Gemisch monoalkylierter, oligomerer oder polymerer Zucker kommt. Bevorzugte Alkylpolyglykoside sind die Alkylpolyglucoside, wobei besonders bevorzugt der Alkohol ein langkettiger Fettalkohol oder ein Gemisch langkettiger Fettalkohole mit verzweigten oder unverzweigten $C_8$- bis $C_{18}$-Alkylketten ist und der Oligomerisierungsgrad (DP) der Zucker zwischen 1 und 10, vorzugsweise 1 bis 6, insbesondere 1,1 bis 3, äußerst bevorzugt 1,1 bis 1,7, beträgt, beispielsweise $C_{8-10}$-Alkyl-1.5-glucosid (DP von 1,5).

Vorzugsweise werden Fettalkoholethoxylate in Mengen von bis zu 20 Gew.-%, besonders bevorzugt 4 bis 12 Gew.-%, besonders bevorzugt 7 bis 9 Gew.-% eingesetzt. Daneben können weitere nichtionische Tenside, etwa Fettsäuremonoalkanolamide und/oder Alkylpolyglykoside, in Mengen von bis zu 10 Gew.-% enthalten sein.

Weitere anionische Tenside

Als weitere anionische Tenside können im erfindungsgemäßen WC-Stein aliphatische Sulfate wie Fettalkoholsulfate, Fettalkoholethersulfate, Dialkylethersulfate, Monoglyceridsulfate und aliphatische Sulfonate wie Alkansulfonate, Ethersulfonate, n-Alkylethersulfonate, Estersulfonate und Ligninsulfonate sein. Ebenfalls im Rahmen der vorliegenden Erfindung verwendbar sind Fettsäurecyanamide, Sulfosuccinate (Sulfobernsteinsäureester), insbesondere Sulfobernsteinsäuremono- und -di-$C_8$-$C_{18}$-Alkylester, Sulfosuccinamate, Sulfosuccinamide, Fettsäureisethionate, Acylaminoalkansulfonate (Fettsäuretauride), Fettsäuresarcosinate, Ethercarbonsäuren und Alkyl(ether)phosphate sowie $\alpha$-Sulfofettsäuresalze, Acylglutamate, Monoglyceriddisulfate und Alkylether des Glycerindisulfats.

Bevorzugt im Rahmen der vorliegenden Erfindung sind die Fettalkoholsulfate und/oder Fettalkoholethersulfate, insbesondere die Fettalkoholsulfate. Fettalkoholsulfate sind Produkte von Sulfatierreaktionen an entsprechenden Alkoholen, während Fettalkoholethersulfate Produkte von Sulfatierreaktionen an alkoxylierten Alkoholen sind. Dabei versteht der Fachmann allgemein unter alkoxylierten Alkoholen die Reaktionsprodukte von Alkylenoxid, bevorzugt Ethylenoxid, mit Alkoholen, im Sinne der vorliegenden Erfindung bevorzugt mit längerkettigen Alkoholen. In der Regel entsteht aus n Molen Ethylenoxid und einem Mol Alkohol, abhängig von den Reaktionsbedingungen, ein komplexes Gemisch von Additionsprodukten unterschiedlicher Ethoxylierungsgrade. Eine weitere Ausführungsform der Alkoxylierung besteht im Einsatz von Gemischen der Alkylenoxide, bevorzugt des Gemisches von Ethylenoxid und Propylenoxid. Bevorzugte Fettalkoholethersulfate sind die Sulfate niederethoxylierter Fettalkohole mit 1 bis 4 Ethylenoxideinheiten (EO), insbesondere 1 bis 2 EO, beispielsweise 1,3 EO.

Die anionischen Tenside werden vorzugsweise als Natriumsalze eingesetzt, können aber auch als andere Alkali- oder Erdalkalimetallsalze, beispielsweise Magnesiumsalze, sowie in Form von Ammonium- oder Mono-, Di, Tri- bzw. Tetraalkylammoniumsalzen enthalten sein, im Falle der Sulfonate auch in Form ihrer korrespondierenden Säure, z.B. Dodecylbenzolsulfonsäure.

Neben den bisher genannten Tensidtypen kann das erfindungsgemäße Mittel weiterhin auch Kationtenside und/oder amphotere Tenside enthalten.

Geeignete Amphotenside sind beispielsweise Betaine der Formel $(R^{iii})(R^{iv})(R^v)N^+CH_2COO^-$, in der $R^{iii}$ einen gegebenenfalls durch Heteroatome oder Heteroatomgruppen unterbrochenen Alkylrest mit 8 bis 25, vorzugsweise 10 bis 21 Kohlenstoffatomen und $R^{iv}$ sowie $R^v$ gleichartige oder verschiedene Alkylreste mit 1 bis 3 Kohlenstoffatomen bedeuten, insbesondere $C_{10}$-$C_{18}$-Alkyl-dimethylcarboxymethylbetain und $C_{11}$-$C_{17}$-Alkylamidopropyl-dimethylcarboxy-methylbetain.

Geeignete Kationtenside sind u.a. die quartären Ammoniumverbindungen der Formel $(R^{vi})(R^{vii})(R^{viii})(R^{ix})N^+$ $X^-$, in der $R^{vi}$ bis $R^{ix}$ für vier gleich- oder verschiedenartige, insbesondere zwei lang- und zwei kurzkettige, Alkylreste und $X^-$ für ein Anion, insbesondere ein Halogenidion, stehen, beispielsweise Didecyl-dimethyl-ammoniumchlorid, Alkyl-benzyl-didecyl-ammoniumchlorid und deren Mischungen.

Weitere Inhaltsstoffe

Neben den bisher genannten Komponenten kann der erfindungsgemäße WC-Stein weitere, üblicherweise in WC-Steine eingesetzte Inhaltsstoffe enthalten, vorzugsweise ausgewählt aus der Gruppe umfassend Säuren, Basen, Salze, Verdickungsmittel, antimikrobielle Wirkstoffe, Konservierungsstoffe, Komplexbildner, Polymere, Farbstoffe, Duftstoffe, Parfümbooster, Füllstoffe, Builder, Bleichmittel, Korrosionsinhibitoren, Abspülregulatoren, Enzyme, Mikroorganismen, Wirkstoffe zur Biofilmentfernung, Wirkstoffe zur Inhibierung der Kalkablagerung, Wirkstoffe zur Verminderung der Schmutzhaftung, Wirkstoffe zur Verbesserung der Verarbeitbarkeit, Wirkstoffe zur Verringerung der Klebrigkeit sowie Gemische derselben. Insgesamt sollten nicht mehr als 60 Gew.-% weitere Inhaltsstoffe enthalten sein, vorzugsweise 0,01 bis 60 Gew.-%, insbesondere 0,2 bis 15 Gew.-%.

Säuren

Erfindungsgemäße WC-Steine können zur Verstärkung der Reinigungsleistung gegenüber Kalk und Urinstein eine oder mehrere Säuren und/oder deren Salze enthalten. Bevorzugt werden die Säuren aus nachwachsenden Rohstoffen hergestellt. Als Säuren eignen sich daher insbesondere organische Säuren wie Ameisensäure, Essigsäure, Citronensäure, Glycolsäure, Milchsäure, Bernsteinsäure, Adipinsäure, Äpfelsäure, Weinsäure und Gluconsäure sowie Gemische derselben. Daneben können aber auch die anorganischen Säuren Salzsäure, Schwefelsäure, Phosphorsäure und Salpetersäure oder auch Amidosulfonsäure bzw. deren Mischungen eingesetzt werden. Besonders bevorzugt sind die Säuren und/oder ihre Salze ausgewählt aus der Gruppe umfassend Citronensäure, Milchsäure, Ameisensäure, ihre Salze sowie Gemische derselben. Sie werden vorzugsweise in Mengen von 0,01 bis 10 Gew.-% eingesetzt, besonders bevorzugt 0,2 bis 5 Gew.-%.

Daneben enthält die Zusammensetzung in einer bevorzugten Ausführungsform anorganische Salze, vorzugsweise Alkali- oder Erdalkalimetallsalze, insbesondere Carbonate, Sulfate, Halogenide oder Phosphate sowie Gemische derselben. Besonders bevorzugt werden Natriumsulfat und/oder Natriumcarbonat eingesetzt. Natriumsulfat kann dabei in einer Menge von bis zu 60 Gew.-% enthalten sein, vorzugsweise 0,01 bis 60 Gew.-%, besonders bevorzugt 20 bis 60 Gew.-%, insbesondere 35 bis 55 Gew.-%. Natriumcarbonat und weitere Salze können in einer Menge von bis zu 30 Gew.-%, vorzugsweise bis zu 10 Gew.-%, besonders bevorzugt bis zu 5 Gew.-% enthalten sein.

Basen

In erfindungsgemäßen Zusammensetzungen können weiterhin Alkalien enthalten sein. Als Basen werden in erfindungsgemäßen Zusammensetzungen vorzugsweise solche aus der Gruppe der Alkali- und Erdalkalimetallhydroxide und -carbonate, insbesondere Natriumcarbonat oder Natriumhydroxid, eingesetzt. Daneben können aber auch Ammoniak und/oder Alkanolamine mit bis zu 9 C-Atomen im Molekül verwendet werden, vorzugsweise die Ethanolamine, insbesondere Monoethanolamin.

Antimikrobielle Wirkstoffe

Eine besondere Form der Reinigung stellen die Desinfektion und die Sanitation dar. In einer entsprechenden besonderen Ausführungsform der Erfindung enthält der WC-Reinigungsblock daher einen oder mehrere antimikrobielle Wirkstoffe, vorzugsweise in einer Menge von 0,01 bis 1 Gew.-%, vorzugsweise 0,02 bis 0,8 Gew.-%, insbesondere 0,05 bis 0,5 Gew.-%, besonders bevorzugt 0,1 bis 0,3 Gew.-%, äußerst bevorzugt 0,2 Gew.-%.

Die Begriffe Desinfektion, Sanitation, antimikrobielle Wirkung und antimikrobieller Wirkstoff haben im Rahmen der erfindungsgemäßen Lehre die fachübliche Bedeutung. Während Desinfektion im engeren Sinne der medizinischen Praxis die Abtötung von - theoretisch allen - Infektionskeimen bedeutet, ist unter Sanitation die möglichst weitgehende Eliminierung aller - auch der für den Menschen normalerweise unschädlichen saprophytischen - Keime zu verstehen. Hierbei ist das Ausmaß der Desinfektion bzw. Sanitation von der antimikrobiellen Wirkung der angewendete Zusammensetzung abhängig, die mit abnehmendem Gehalt an antimikrobiellem Wirkstoff bzw. zunehmender Verdünnung der Zusammensetzung zur Anwendung abnimmt.

Erfindungsgemäß geeignet sind beispielsweise antimikrobielle Wirkstoffe aus den Gruppen der Alkohole, Aldehyde, antimikrobiellen Säuren bzw. deren Salze, Carbonsäureester, Säureamide, Phenole, Phenolderivate, Diphenyle, Diphenylalkane, Harnstoffderivate, Sauerstoff-, Stickstoff-Acetale sowie -Formale, Benzamidine, Isothiazole und deren Derivate wie Isothiazoline und Isothiazolinone, Phthalimidderivate, Pyridinderivate, antimikrobiellen oberflächenaktiven Verbindungen, Guanidine, antimikrobiellen amphoteren Verbindungen, Chinoline, 1,2-Dibrom-2,4-dicyanobutan, Iodo-2-propynyl-butyl-carbamat, Iod, Iodophore, Aktivchlor abspaltenden Verbindungen und Peroxide. Bevorzugte antimikrobielle Wirkstoffe werden vorzugsweise ausgewählt aus der Gruppe umfassend Ethanol, n-Propanol, i-Propanol, 1,3-Butandiol, Phenoxyethanol, 1,2-Propylenglykol, Glycerin, Undecylensäure, Citronensäure, Milchsäure, Benzoesäure, Salicylsäure, Thymol, 2-Benzyl-4-chlorphenol, 2,2'-Methylen-bis-(6-brom-4-chlorphenol), 2,4,4'-Trichlor-2'-hydroxydiphenylether, N-(4-Chlorphenyl)-N-(3,4-dichlorphenyl)-harnstoff, N,N'-(1,10-decandiyldi-1-pyridinyl-4-yliden)-bis-(1-octanamin)-dihydrochlorid, N,N'-Bis-(4-Chlorphenyl)-3,12-diimino-2,4,11,13-tetraazatetradecandiimidamid, antimikrobielle quaternäre oberflächenaktive Verbindungen, Guanidine und Natrium-Dichlorisocyanurat (DCI, 1,3-Dichlor-5H-1,3,5-triazin-2,4,6-trion Natriumsalz). Bevorzugte antimikrobiell wirkende oberflächenaktive quaternäre Verbindungen enthalten eine Ammonium-, Sulfonium-, Phosphonium-, Jodonium- oder Arsoniumgruppe. Weiterhin können auch antimikrobiell wirksame ätherische Öle eingesetzt werden, die gleichzeitig für eine Beduftung des Reinigugsmittels sorgen. Besonders bevorzugte antimikrobielle Wirkstoffe sind jedoch ausgewählt aus der Gruppe umfassend Salicylsäure, quaternäre Tenside, insbesondere Benzalkoniumchlorid, Peroxo-Verbindungen, insbesondere Wasserstoffperoxid, Alkalimetallhypochlorit, Natriumdichlorisocyanurat sowie Gemische derselben.

Konservierungsstoffe

Konservierungsstoffe können gleichfalls in erfindungsgemäßen WC-Reinigungsblöcken enthalten sein. Als solche können im Wesentlichen die bei den antimikrobiellen Wirkstoffen genannten Stoffe eingesetzt werden.

Komplexbildner

Komplexbildner (*INCI* Chelating Agents), auch Sequestriermittel genannt, sind Inhaltsstoffe, die Metallionen zu komplexieren und inaktivieren vermögen, um ihre nachteiligen Wirkungen auf die Stabilität oder das Aussehen der Mittel, beispielsweise Trübungen, zu verhindern. Einerseits ist es dabei wichtig, die mit zahlreichen Inhaltsstoffen inkompatiblen Calcium- und Magnesiumionen der Wasserhärte zu komplexieren. Die Komplexierung der Ionen von Schwermetallen wie Eisen oder Kupfer verzögert andererseits die oxidative Zersetzung der fertigen Mittel. Zudem unterstützen die Komplexbildner die Reinigungswirkung.

Geeignet sind beispielsweise die folgenden gemäß *INCI* bezeichneten Komplexbildner: Aminotrimethylene Phosphonic Acid, Beta-Alanine Diacetic Acid, Calcium Disodium EDTA, Citric Acid, Cyclodextrin, Cyclohexanediamine Tetraacetic Acid, Diammonium Citrate, Diammonium EDTA, Diethylenetriamine Pentamethylene Phosphonic Acid, Dipotassium EDTA, Disodium Azacycloheptane Diphosphonate, Disodium EDTA, Disodium Pyrophosphate, EDTA, Etidronic Acid, Galactaric Acid, Gluconic Acid, Glucuronic Acid, HEDTA, Hydroxypropyl Cyclodextrin, Methyl Cyclodextrin, Pentapotassium Triphosphate, Pentasodium Aminotrimethylene Phosphonate, Pentasodium Ethylenediamine Tetramethylene Phosphonate, Pentasodium Pentetate, Pentasodium Triphosphate, Pentetic Acid, Phytic Acid, Potassium Citrate, Potassium EDTMP, Potassium Gluconate, Potassium Polyphosphate, Potassium Trisphosphonomethylamine Oxide, Ribonic Acid, Sodium Chitosan Methylene Phosphonate, Sodium Citrate, Sodium Diethylenetriamine Pentamethylene Phosphonate, Sodium Dihydroxyethylglycinate, Sodium EDTMP, Sodium Gluceptate, Sodium Gluconate, Sodium Glycereth-1 Polyphosphate, Sodium Hexametaphosphate, Sodium Metaphosphate, Sodium Metasilicate, Sodium Phytate, Sodium Polydimethylglycinophenolsulfonate, Sodium Trimetaphosphate, TEA-EDTA, TEA-Polyphosphate, Tetrahydroxyethyl Ethylenediamine, Tetrahydroxypropyl Ethylenediamine, Tetrapotassium Etidronate, Tetrapotassium Pyrophosphate, Tetrasodium EDTA, Tetrasodium Etidronate, Tetrasodium Pyrophosphate, Tripotassium EDTA, Trisodium Dicarboxymethyl Alaninate, Trisodium EDTA, Trisodium HEDTA, Trisodium NTA und Trisodium Phosphate.

Polymere

Der erfindungsgemäße WC-Stein kann weiterhin Polymere enthalten. Diese können beispielsweise zur Verringerung der Kalkbildung sowie der Wiederanschmutzungsneigung dienen.

Bevorzugte Polymere sind dabei Acrylpolymere, wie sie etwa von der Firma Rhodia unter dem Handelsnamen Mirapol kommerziell erhältlich sind.

Duft- und Farbstoffe

Als weitere Inhaltsstoffe kann der erfindungsgemäße WC-Stein einen oder mehrere Duftstoffe und/oder ein oder mehrere Farbstoffe (*INCI* Colorants) enthalten. Als Farbstoffe können dabei sowohl wasserlösliche als auch öllösliche Farbstoffe verwendet werden, wobei einerseits die Kompatibilität mit weiteren Inhaltsstoffen, beispielsweise Bleichmitteln, zu beachten ist und andererseits der eingesetzte Farbstoff gegenüber der WC-Keramik auch bei längerem Einwirken nicht substantiv wirken sollte. Die Farbstoffe sind vorzugsweise in einer Menge von 0,0001 bis 0,1 Gew.-%, insbesondere 0,0005 bis 0,05 Gew.-%, besonders bevorzugt 0,001 bis 0,01 Gew.-%, enthalten.

Builder

In den erfindungsgemäßen WC-Stein können ggf. wasserlösliche und/oder wasserunlösliche Builder eingesetzt werden. Dabei sind wasserlösliche Builder bevorzugt, da sie in der Regel weniger dazu tendieren, auf harten Oberflächen unlösliche Rückstände zu hinterlassen. Übliche Builder, die im Rahmen der Erfindung zugegen sein können, sind die niedermolekularen Polycarbonsäuren und ihre Salze, die homopolymeren und copolymeren Polycarbonsäuren und ihre Salze, die Citronensäure und ihre Salze, die Carbonate, Phosphate und Silikate. Zu wasserunlöslichen Buildern zählen die Zeolithe, die ebenfalls verwendet werden können, ebenso wie Mischungen der vorgenannten Buildersubstanzen.

Bleichmittel

Erfindungsgemäß können Bleichmittel dem Reinigungsmittel zugesetzt werden. Geeignete Bleichmittel umfassen Peroxide, Persäuren und/oder Perborate, besonders bevorzugt ist Wasserstoffperoxid. Natriumhypochlorit ist dagegen bei sauer formulierten Reinigungsmitteln aufgrund der Freisetzung giftiger Chlorgas-Dämpfe weniger geeignet, kann jedoch in alkalisch eingestellten Reinigungsmitteln eingesetzt werden. Unter Umständen kann neben dem Bleichmittel auch ein Bleichaktivator vonnöten sein.

Korrosionsinhibitoren

Geeignete Korrosionsinhibitoren (*INCI* Corrosion Inhibitors) sind beispielsweise folgende gemäß *INCI* benannte Substanzen: Cyclohexylamine, Diammonium Phosphate, Dilithium Oxalate, Dimethylamino Methylpropanol, Dipotassium Oxalate, Dipotassium Phosphate, Disodium Phosphate, Disodium Pyrophosphate, Disodium Tetrapropenyl Succinate, Hexoxyethyl Diethylammonium, Phosphate, Nitromethane, Potassium Silicate, Sodium Aluminate, Sodium Hexametaphosphate, Sodium Metasilicate, Sodium Molybdate, Sodium Nitrite, Sodium Oxalate, Sodium Silicate, Stearamidopropyl Dimethicone, Tetrapotassium Pyrophosphate, Tetrasodium Pyrophosphate, Triisopropanolamine.

Abspülregulatoren

Die als Abspülregulatoren bezeichneten Substanzen dienen in erster Linie dazu, den Verbrauch der Zusammensetzung während des Einsatzes so zu steuern, dass die vorgesehene Standzeit eingehalten wird. Als Regulatoren eignen sich vorzugsweise feste langkettige Fettsäuren, wie Stearinsäure, aber auch Salze solcher Fettsäuren, Fettsäureethanolamide, wie Kokosfettsäuremonoethanolamid, oder feste Polyethylenglykole, wie solche mit Molekulargewichten zwischen 10000 und 50000.

Wirkstoffe zur Verringerung der Klebrigkeit

Zur Verbesserung der Verarbeitbarkeit bei der Herstellung des erfindungsgemäßen WC-Steins kann ein Wirkstoff zur Verringerung der Klebrigkeit zugesetzt werden. So verbessert die Zugabe von Dolomitpulver oder Titandioxidpulver mit feiner Partikelgrößenverteilung das Verarbeitungsverhalten beim Kugelformen und reduziert deutlich Abrieb bzw. Klebrigkeit.

Die Ergebnisse mit solchen Wirkstoffen sind besser als mit anderen üblichen Maßnahmen, beispielsweise Beschichten der Kugeln mit einem Gleitmittel, Abpudern oder Beschichten der Formwalzen mit Teflon.

Enzyme

Die Zusammensetzung kann auch Enzyme enthalten, vorzugsweise Proteasen, Lipasen, Amylasen, Hydrolasen und/oder Cellulasen. Sie können der erfindungsgemäßen Zusammensetzung in jeder nach dem Stand der Technik etablierten Form Enzyme zugesetzt werden. Hierzu gehören Lösungen der Enzyme, vorteilhafterweise möglichst konzentriert, wasserarm und/oder mit Stabilisatoren versetzt. Alternativ können die Enzyme verkapselt werden, beispielsweise durch Sprühtrocknung oder Extrusion der Enzymlösung zusammen mit einem, vorzugsweise natürlichen, Polymer oder in Form von Kapseln, beispielsweise solchen, bei denen die Enzyme wie in einem erstarrten Gel eingeschlossen sind oder in solchen vom Kern-Schale-Typ, bei dem ein enzymhaltiger Kern mit einer Wasser-, Luft- und/oder Chemikalienundurchlässigen Schutzschicht überzogen ist. In aufgelagerten Schichten können zusätzlich weitere Wirkstoffe, beispielsweise Stabilisatoren, Emulgatoren, Pigmente, Bleich- oder Farbstoffe aufgebracht werden. Derartige Kapseln werden nach an sich bekannten Methoden, beispielsweise durch Schüttel- oder Rollgranulation oder in Fluid-bed-Prozessen aufgebracht. Vorteilhafterweise sind derartige Granulate, beispielsweise durch Aufbringen polymerer Filmbildner, staubarm und aufgrund der Beschichtung lagerstabil.

Weiterhin können in enzymhaltigen Zusammensetzungen Enzymstabilisatoren vorhanden sein, um ein in einer erfindungsgemäße Zusammensetzung enthaltenes Enzym vor Schädigungen wie beispielsweise Inaktivierung, Denaturierung oder Zerfall etwa durch physikalische Einflüsse, Oxidation oder proteolytische Spaltung zu schützen. Als Enzymstabilisatoren sind, jeweils in Abhängigkeit vom verwendeten Enzym, insbesondere geeignet: Benzamidin-Hydrochlorid, Borax, Borsäuren, Boronsäuren oder deren Salze oder Ester, vor allem Derivate mit aromatischen Gruppen, etwa substituierte Phenylboronsäuren beziehungsweise deren Salze oder Ester; Peptidaldehyde (Oligopeptide mit reduziertem C-Terminus), Aminoalkohole wie Mono-, Di-, Triethanol- und -Propanolamin und deren Mischungen, aliphatische Carbonsäuren bis zu $C_{12}$, wie Bernsteinsäure, andere Dicarbonsäuren oder Salze der genannten Säuren; endgruppenverschlossene Fettsäureamidalkoxylate; niedere aliphatische Alkohole und vor allem Polyole, beispielsweise Glycerin, Ethylenglykol, Propylenglykol oder Sorbit; sowie Reduktionsmittel und Antioxidantien wie Natrium-Sulfit und reduzierende Zucker. Weitere geeignete Stabilisatoren sind aus dem Stand der Technik bekannt. Bevorzugt werden Kombinationen von Stabilisatoren verwendet, beispielsweise die Kombination aus Polyolen, Borsäure und/oder Borax, die Kombination von Borsäure oder Borat, reduzierenden Salzen und Bernsteinsäure oder anderen Dicarbonsäuren oder die Kombination von Borsäure oder Borat mit Polyolen oder Polyaminoverbindungen und mit reduzierenden Salzen.

In einer besonders bevorzugten Ausführung der Erfindung, weisen Kern und Mantel unterschiedliche Farbe auf. Weiterhin, besonders bevorzugt, weisen Kern und Mantel jeweils eine homogene Farbe auf. Der Farbunterschied zwischen der Farbe des Mantels und des Kerns $\left(\Delta E_{ab}^{*}\right)$, ist vorzugsweise größer als 3, in dem CIE 1976 (L,a*,b*) Farbkoordinatensystem.

$$\Delta E_{ab}^{*} = \sqrt{(L_2^* - L_1^*)^2 + (a_2^* - a_1^*)^2 + (b_2^* - b_1^*)^2}$$

Alternativ, oder ergänzend, wird es bevorzugt, dass mindestens eine Farbe, vorzugsweise beide, des Kerns und des Mantels, eine Buntheit (Chroma) zwischen 20% und 99%, vorzugsweise zwischen 30% und 95% aufweist.

Durch die erfindungsgemäßen Farbeinstellungen, einzeln oder vorzugsweise kombiniert, wird es dem Verbraucher des WC-Steins eine Erkennung des Verbrauchsgrades stark verbessert. Da die Kontrastwirkung der Elemente untereinander die Beobachtung einfacher machen.

In einer alternative Variante der Erfindung, ist der Farbunterschied zwischen der Farbe des Mantels und des Kerns $\left(\Delta E_{ab}^{*}\right)$, vorzugsweise kleiner als 10, vorzugsweise kleiner als 3, in dem CIE 1976 (L,a*,b*) Farbkoordinatensystem. In dem Fall ist es bevorzugt, dass der Kern unterschiedliche Wirkstoffe und/oder unterschiedliche Konzentration eines gleichen Wirkstoffes zum Mantel hat. Dem Verbraucher fällt es nicht auf, wenn der Mantel Verbraucht ist, und es kann den Eindruck entstehen, dass der WC-Stein aus einer einzigen Wirkstoffzusammensetzung besteht. Trotzdem kann z.B. die Funktionalität und die Wirkung des WC-Steins bei starker Abnutzung durch eine geänderte Wirkstoffzusammensetzung des Kerns mindestens teilweise kompensiert werden.

In der vorliegenden Beschreibung, beziehen sich die Farb-Merkmale auf dem CIE1976 Farbkoordinatensystem. Falls ein Illuminat nötig ist, wird er D65, oder eine wie üblich anwendbare Näherung dessen, verwendet.

Vorrichtung

Die vorliegende Erfindung sieh auch eine Vorrichtung vor, die einen WC-Stein, wie hierin beschrieben, umfasst und ein Haltemittel um den WC-Stein an dem Toilettenbecken zu halten. Vorzugsweise ist die Vorrichtung ein WC-Körbchen.

WC-Körbchen

Erfindungsgemäß wird ein WC-Körbchen vorgesehen, umfassend mindestens einen erfindungsgemäßen WC-Stein. Das WC-Körbchen kann vorzugsweise auch mindestens zwei, besonders bevorzugt mindestens drei erfindungsgemäße WC-Steine umfassen. Die WC-Steine umfassen jeweils mindestens eine Polkappe, und sind untereinander regelmäßig oder unregelmäßig angeordnet. Bevorzugt ist die unregelmäßige Anordnung wegen der vereinfachten Herstellmethode. Die regelmäßige Anordnung ist aber auch interessant, insbesondere der ästhetischen Form wegen, vor allem wenn Pol und Mantel eine unterschiedliche Farbe aufweisen. Die WC-Steine des WC-Körbchens sind rotationssymmetrisch, vorzugsweise kugelförmig.

Das erfindungsgemäße WC-Körbchen ist geeignet zur Abgabe von Zusammensetzungen in ein Toilettenbecken. Es umfasst einen im Toilettenbecken anzuordnenden Aufnahmebehälter, wobei der Aufnahmebehälter am Toilettenbecken befestigbar ist. Der Aufnahmebehälter umfasst eine erste Kammer, die mindestens einen WC-Stein aufnimmt. Vorzugsweise umfasst der Aufnahmebehälter weiter eine zweite Kammer, die einen zweiten WC-Stein aufnimmt. Vorzugsweise, ist mindestens ein, vorzugsweise jeder, der WC-Steine durch Stützmittel vom Boden der Kammer getrennt. Mindestens eine, vorzugsweise alle Kammern, geben bei Überströmung oder Durchströmung mit Spülwasser jeweils die Zusammensetzungen in das Toilettenbecken ab. Das WC-Körbchen ist derart gestaltet, dass bei Überströmung von Spülwasser, das Spülwasser auch in Kontakt mit dem WC-Stein kommt und, dass das mit Wirkstoffzubereitung angereichertes Spülwasser das WC-Körbchen austreten kann. Dazu können z.B. Ein- und Auslassöffnungen, Wasserverteilelemente, und andere Mittel vorgesehen sein. Ausführungsbeispiele werden Anhand der Figuren beschrieben.

Vorzugsweise ist das WC-Körbchen mit dem(den) WC-Stein(ne) unöffenbar verschlossen. Somit, ist es nicht möglich ohne erheblichen Kraftaufwand und Deformierung des WC-Körbchens an den(die) WC-Stein(e) heranzukommen.

Vorzugsweise ist das WC-Körbchen transparent. Damit kann ein Verbraucher nicht nur die Farbe erkennen sondern auch die Form des WC-Steins.

Figurenliste

Fig.1 zeigt einen erfindungsgemäßen WC-Stein in Draufsicht auf den Pol;
Fig.2 zeigt einen erfindungsgemäßen WC-Stein in Seitenansicht;

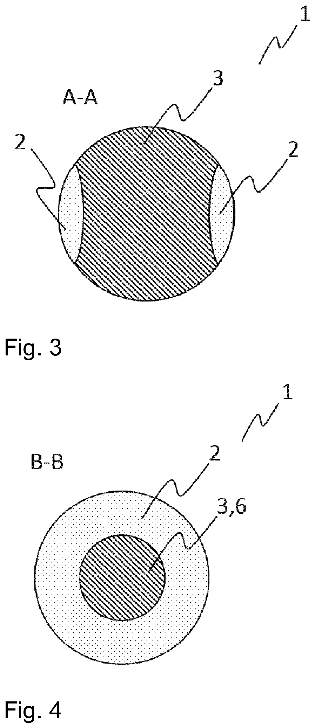

Fig.3 zeigt einen Schnitt des WC-Steins aus Fig. 2 in Richtung A-A.
Fig.4 zeigt einen Schnitt des WC-Steins aus Fig. 2 in Richtung B-B.
Fig.5 zeigt im Querschnitt das Pressverfahren bevor und nach dem Pressen.
Fig.6 zeigt einen Schnitt eines WC-Körbchens umfassend 3 WC-Steine.

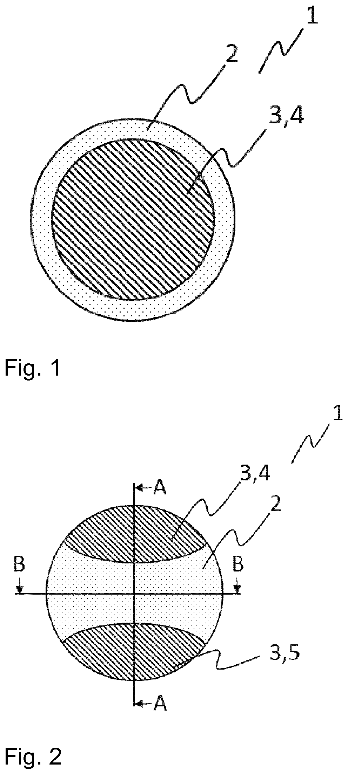

In einem besonderen bevorzugten Ausführungsbeispiel, umfasst der WC-Stein 1 den Mantel 2 und den Kern 3. Der Kern 3 formt einen Pol 4 der in der Draufsicht gemäß Fig. 1 sichtbar ist. In der Seitenansicht gemäß Fig. 2 sind der erste Pol 4 und der zweite Pol 5 sichtbar. Der Kern 3 wird von dem Mantel 2 umschlossen. Der Mantel 2 ist ringförmig, wie in der Schnittansicht A-A mit beispielsweise einem annähernd elliptischen Schnitt und in der Schnittansicht B-B als kreisförmiger Ring zu sehen ist. Der Pol 4 und/oder der Pol 5 können/kann jeweils mehr oder weniger ausgeprägt sein. Dadurch wird eine vordefinierte, gesteuerte Freisetzung von Wirkstoffen über den zeitlichen Verlauf ermöglicht. Auch der Durchmesser des Segments 6 kann geringer oder größer sein. Statt eines zylindrischen Abschnitts des Segments 6, kann dieses auch eine andere Geometrie aufweisen.

Vergleichsbeispiel

WC-Steine wurden mit folgender Zusammensetzung hergestellt und als Referenz verwendet:

|  | E1 |
| --- | --- |
| $C_{10-13}$-lin. Alkylbenzolsulfonat-Na | 26 |
| Fettalkoholsulfat-Na | -- |
| $C_{12}$- Fettalkoholsulfat-Na | - |
| $C_{14-16}$-Olefinsulfonat-Na | 18 |
| $C_{16-18}$-Fettalkoholethoxylat 25 EO | 8 |
| Cellulose | -- |
| Trinatriumcitrat-Dihydrat | 1 |

(fortgesetzt)

|  | E1 |
|---|---|
| Natriumsulfat | ad 100 |
| Natriumcarbonat | -- |
| $C_{12-18}$-Fettsäuremonoethanolamid | -- |
| Natriumsilikat | -- |
| Parfüm | 4,5 |
| Farbstoff | + |

Die verwendete Parfümzusammensetzung wurde wie folgt zusammengesetzt:

| Dosage Gew.-% | Name | CAS Nr. |
|---|---|---|
| 12,16 | dipropylengjycol | 25265-71-8 |
| 8,51 | dihydromyrcenol | 18479-58-8 |
| 7,30 | terpineol | 8000-41-7 |
| 4,86 | linalylacetat | 115-95-7 |
| 3,65 | citronenoel messina | 84929-31-7 |
| 3,65 | agrunitril | 51566-62-2 |
| 3,65 | orangenoel suess ital. | 8028-48-6 |
| 3,65 | otbca | 88-41-5 |
| 3,65 | styrolylacetat | 93-92-5 |
| 3,65 | linalool | 78-70-6 |
| 2,43 | aldehyd c 08 | 124-13-0 |
| 2,43 | alkohol c 08 | 111-87-5 |
| 2,43 | allylamylglycolat | 67634-00-8 |
| 2,43 | benzylacetat | 140-11-4 |
| 2,43 | hedione | 24851-98-7 |
| 1,52 | linalool oxyde | 1365-19-1 |
| 1,52 | lemonile | 61792-11-8 |
| 1,52 | bromelia | 93-18-5 |
| 1,52 | yara yara | 93-04-9 |
| 1,52 | aldehyd c 10 | 112-31-2 |
| 1,51 | aldehyd c 14 sog | 104-67-6 |
| 1,50 | benzylaceton | 2550-26-7 |
| 1,50 | acedyl | 54830-99-8 |
| 1,50 | propidyl | 68912-13-0 |
| 1,50 | isobornylacetat | 125-12-2 |
| 1,50 | terpinolen 30 | 586-62-9 |
| 1,50 | lilial, lysmeral | 80-54-6 |
| 1,50 | citronellol rein | 106-22-9 |

(fortgesetzt)

| Dosage Gew.-% | Name | CAS Nr. |
|---|---|---|
| 1,50 | geraniol rein | 106-24-1 |
| 1,50 | nerol standard | 106-25-2 |
| 1,50 | phenylethylalkohol | 60-12-8 |
| 1,50 | geranylacetat | 16409-44-2 |
| 1,50 | hexylzimtaldehyd (alpha) | 101-86-0 |
| 1,50 | isoraldein 70 | 1335-46-2 |
| 1,50 | jonon beta synth. | 14901-07-6 |
| 1,50 | iso e super | 54464-57-2 |
| 1,50 | ethylenbrassilat | 105-95-3 |

Die Zusammensetzung wurde gemischt, anschließend zu einem Strang extrudiert, geschnitten und zu Kugeln verformt, in einer Form die. Der Enddurchschnitt jeder Kugel betrug 25,4 mm.

Beispiel

Erfindungsgemäße WC-Steine wurden wie folgt hergestellt. Eine erste Zusammensetzung A wurde bereitgestellt wie bei dem Referenzbeispiel, mit dem einzigen Unterschied, dass die Parfümkonzentration von 4 +/- 0.5 auf 5,5 +/- 0.5 geändert wurde. Eine zweite Zusammensetzung B wurde bereitgestellt wie bei dem Referenzbeispiel, mit dem einzigen Unterschied, dass die Parfümkonzentration von 4 +/- 0.5 auf 2,5 +/- 0.5 geändert wurde. Wie im Vergleichsbeispiel, wurden die Zusammensetzungen jeweils gemischt. Die Mischung wurde dann in einen koaxialen Strang extrudiert mit Zusammensetzung A als innerem Strang und Zusammensetzung B als äußerem Strang. Dazu wurde ein Extruder verwendet. Die koaxialen, zylindrischen Stränge, hatten einen äußeren Durchmesser von 20,5mm und einen Durchmesser des inneren Strangs von 7,3 mm. Der Strang wurde in 26,36mm lange Zylinder geschnitten, wobei die Zylinder jeweils zu einer Kugel geformt wurden. Der Enddurchmesser jeder Kugel betrug 25,4 mm.

Vergleichsversuche

Kugeln aus dem Beispiel und aus dem Vergleichsbeispiel wurden in identischen WC-Körbchen eingesetzt, und jeweils in einer Testkammer gespült. Die Testkammern haben einen identischen Aufbau, und sind voneinander getrennt, so dass es kein Luftwechsel zwischen den Kammern gibt. Jede Testkammer hat zudem eine WC-Schüssel, und wird durch einen konstanten Luftstrom gespült. Die Testkammer stehen in einem speziell für die Testkammer eingerichteten Raum (Messraum), der geruchsneutral ist, gut belüftet werden kann und gegebenenfalls über einen Aktivkohlefilter zwangsbelüftet werden kann. Der Volumenanteil an Kohlendioxid im Messraum beträgt weniger als 0.15 Vol.-%, die Luftwechselrate der Kammer beträgt mindestens 4.4 m$^3$/h pro Person. Die Temperatur im Messraum beträgt 20 °C und ist während der Messung konstant. Der Messraum ist keiner direkten Sonneneinstrahlung ausgesetzt und auch andere störende Licht- und Geräuschquellen wurden soweit wie möglich minimiert. Alle Vorrichtungen die zur Testkammer gehören müssen geruchlos sein. Die Bewertung der Geruchsstärke wird von mindestens 20 Prüfern vorgenommen, wobei in der Regel gleich viele geruchlich geschulte Männer und Frauen eingesetzt werden, die alle mindestens 16 Jahre alt sind. Die Prüfer sind zum Zeitpunkt der Bewertung der Verbindungen nicht durch störende Faktoren wie Kontakt mit Parfüms, Lebensmitteln, sonstigen Genussmitteln oder auch durch eine Erkältungen oder Allergie beeinflusst.

Die WC-Körbchen wurden in den WC-Schüsseln in identischer Position eingehängt, und jede WC-Schüssel wurde a mal pro Stunde gespült. Jede Spülung verwendete 6 L Wasser. Die Messungen wurden von den Prüfern bei 4, 72, 96, 120, 144, 168 und 172 Stunden durchgeführt. Für die Bewertung macht der Prüfer das Fenster auf, positioniert sein Kopf im Innenraum der Testkammer, führt die Riechprobe durch, zieht seinen Kopf wieder heraus und schließt anschließend das Fenster.

Die Prüfer hatten einen eindeutigen Eindruck, dass die Duftstärke des erfindungsgemäßen Beispiels größer als beim Vergleichsbeispiel war. Der Verbraucher nimmt wahr, dass sich schon nach 72 Stunden der Dufteindruck, im Vergleich zum Vergleichsbeispiel, verstärkt hat. Da der Kern exponiert war, war der Anfangsunterschied zu dem Vergleichsbeispiel gering ausgefallen, lediglich ein Verlust von 24% wurde von dem Verbraucher am Anfang war genommen, der aber überraschend schnell aufgeholt wurde. Überraschenderweise, wahren die Verbraucherergebnisse so, dass die erfindungsgemäße WC-Steine und WC-Körbchen einen stärkeren Dufteindruck über die gesamte Verwendungszeit abgeben als die Referenz, obwohl die verwendete Parfümmenge identisch ist.

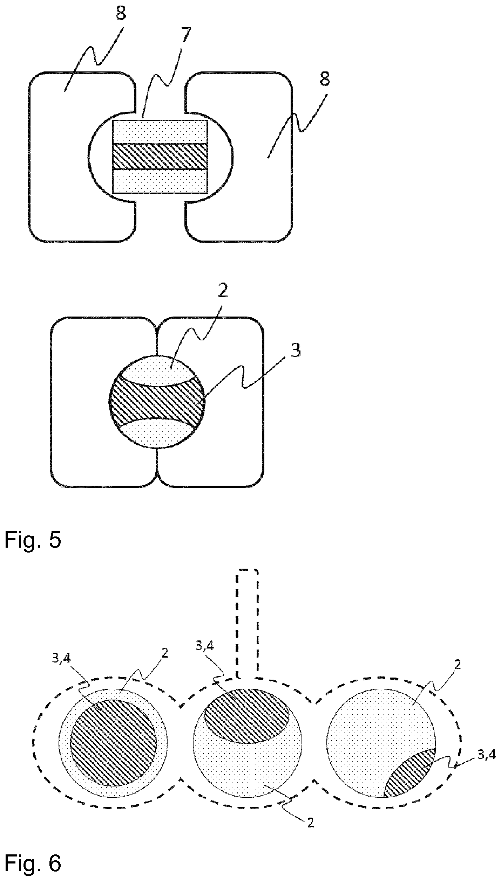

Der wesentliche Teil des erfindungsgemäßen Verfahrens wird, im Querschnitt, in Fig.5 gezeigt. Ein co-extrudierter Strangabschnitt 7 wird in einer Presse 8 platziert. Die Presse 8 hat als Endform eine Kugel, mit identischen Volumen wie der Strangabschnitt 7. Dadurch dass die Mulden der Presse 8 zueinander gebracht werden und somit die Endform erreicht wird, wird der Strangabschnitt zu einer erfindungsgemäßen Kugel verformt.

Ein beispielhaftes erfindungskonformes WC-Körbchen wird in Fig. 6 dargestellt. In Fig. 6 sind 3 WC-Steine gesehen, die jeweils einen Mantel 2 und einen Pol 3 aufweisen. Die WC-Steine sind in dem WC-Körbchen, das gestrichelt mit einem Hänger, von der Vordersicht dargestellt wird, dargestellt. Das Körbchen ist schematisch dargestellt damit die WC-Steine besser zu erkennen sind. Die WC-Steine sind unregelmäßig zueinander angeordnet, da jeder Pol 4 in eine andere Richtung hinweist.

## Patentansprüche

1. WC-Stein (1), der aus mindestens einem Mantel (2) und einen Kern (3) besteht,

    wobei der Mantel (2) eine erste Zusammensetzung umfasst und der Kern (3) eine zweite Zusammensetzung umfasst,
    wobei sich vorzugsweise die erste und die zweite Zusammensetzung unterscheiden durch mindestens eines der folgenden Merkmale: unterschiedliche Wirkstoffe, unterschiedliche Konzentration eines gleichen Wirkstoffes, unterschiedliche Viskosität;
    wobei der Mantel (2) teilweise den Kern (3) umschließt,
    wobei der Kern (3) auf zwei Seiten des WC-Steins, jeweils als ein Pol (4) bis zur Oberfläche ragt und dabei exponiert ist
    **dadurch gekennzeichnet, dass**
    der WC-Stein Kugelförmig ist, und
    der zweite Pol (5) auf der zum ersten Pol (4) gegenüberliegende Seite des WC-Steins (1) zur Oberfläche ragt und dabei exponiert ist, wobei der Mantel (2) den Kern (3) ringförmig ummantelt, und wobei der Verlauf des Kerns (3) derart ausgebildet ist, dass sich die Oberfläche der exponierten Fläche sich beim gleichmäßigen Abtragen der Oberfläche des WC-Steins (1), verändert.

2. WC-Stein (1) nach Anspruch 1, wobei sich die Oberfläche der exponierten Fläche des Kerns (3), beim gleichmäßigen Abtragen der Oberfläche des WC-Steins (1) vergrößert

3. WC-Stein (1) nach Anspruch 1, wobei sich die Oberfläche der exponierten Fläche des Kerns (3) sich, beim gleichmäßigen Abtragen der Oberfläche des WC-Steins (1) verkleinert.

4. WC-Stein (1) nach Anspruch 3, wobei das Flächenverhältnis der exponierten Oberfläche des Kerns (3) zur exponierten Oberfläche des Mantels (2), bei dem ungelösten WC-Stein (1) zwischen 5% und 67% ist, weiter bevorzugt zwischen 10% und 38%.

5. WC-Stein (1) nach Anspruch 3 und/oder 4, wobei das Flächenverhältnis der exponierten Oberfläche des Kerns (3) zur exponierten Oberfläche des Mantels (2), beim teilweise verbrauchten WC-Stein (1) zwischen 1:3 bis 1:1 ist.

6. WC-Stein (1) nach mindestens einem der vorangehenden Ansprüche, wobei mindestens ein, vorzugsweise der mittlere Abschnitt des Kerns (3), als ein zylindrisches Segment (6) gestaltet ist.

7. WC-Stein (1) nach mindestens einem der vorangehenden Ansprüche, wobei der erste Pol (4) und der zweite Pol (5) durch einen strangförmigen Kern (3) verbunden sind.

8. WC-Stein (1) nach mindestens einem der vorangehenden Ansprüche, wobei der Querschnittsverlauf des Kerns (3) zwischen erstem Pol (4) und zweitem Pol (5) eine Querschnittsverkleinerung aufweist.

9. WC-Stein (1) nach mindestens einem der Ansprüche 1-7, wobei der Querschnittsverlauf des Kerns (3) zwischen erstem Pol (4) und zweitem Pol (5) eine Querschnittsvergrößerung aufweist.

10. WC-Stein (1) nach mindestens einem der vorangehenden Ansprüche, wobei der WC-Stein (1) jeweils eine Sphärizität zwischen 0,8 und 1 aufweist.

**11.** WC-Stein (1) nach mindestens einem der vorangehenden Ansprüche, wobei die erste und die zweite Zusammensetzung sich durch eine unterschiedliche Konzentration eines gleichen Wirkstoffes unterscheiden, wobei der Kern (3) eine höhere Konzentration eines Wirkstoffes von mindestens 0,5 Gew.-%, vorzugsweise mindestens 1 Gew.-% bis 10 Gew.-% aufweist.

**12.** WC-Körbchen mit mindestens zwei, vorzugsweise mindestens drei WC-Steinen (1), nach mindestens einem der vorangehenden Ansprüche, wobei die WC-Steine (1), mit jeweils mindestens einem Pol (4, 5) untereinander regelmäßig oder unregelmäßig angeordnet sind.

**13.** WC-Körbchen nach Anspruch 12, wobei die WC-Steine (1) unregelmäßig zueinander angeordnet sind.

**Claims**

**1.** A toilet rim block (1) consisting of at least a casing (2) and a core (3), the casing (2) comprising a first composition and the core (3) comprising a second composition,

the first and the second composition preferably differing by at least one of the following features: different active ingredients, different concentration of the same active ingredient, different viscosity;
the casing (2) surrounding the core (3) in part,
the core (3) projecting on two sides of the toilet rim block, in each case as a pole (4), as far as the surface and thus being exposed,
**characterized in that**
the toilet rim block is spherical, and
the second pole (5) projects to the surface on the side of the toilet rim block (1) opposite the first pole (4), and is thus exposed, the casing (2) surrounding the core (3) in an annular manner, and the profile of the core (3) being designed such that the surface of the exposed face changes with uniform wear of the surface of the toilet rim block (1).

**2.** The toilet rim block (1) according to claim 1, wherein the surface of the exposed face of the core (3) increases with uniform wear of the surface of the toilet rim block (1).

**3.** The toilet rim block (1) according to claim 1, wherein the surface of the exposed face of the core (3) decreases with uniform wear of the surface of the toilet rim block (1).

**4.** The toilet rim block (1) according to claim 3, wherein, in the case of the undissolved toilet rim block (1), the area ratio of the exposed surface of the core (3) to the exposed surface of the casing (2) is between 5% and 67%, more preferably between 10% and 38%.

**5.** The toilet rim block (1) according to claim 3 and/or claim 4, wherein, when the toilet rim block (1) has been partially used, the area ratio of the exposed surface of the core (3) to the exposed surface of the casing (2) is between 1:3 and 1:1.

**6.** The toilet rim block (1) according to at least one of the preceding claims, wherein at least one portion, preferably the central portion, of the core (3) is a cylindrical segment (6).

**7.** The toilet rim block (1) according to at least one of the preceding claims, wherein the first pole (4) and the second pole (5) are connected by a strand-like core (3).

**8.** The toilet rim block (1) according to at least one of the preceding claims, wherein the cross-sectional profile of the core (3) has a reduction in cross section between the first pole (4) and the second pole (5).

**9.** The toilet rim block (1) according to at least one of the preceding claims 1-7, wherein the cross-sectional profile of the core (3) has an enlargement in cross section between the first pole (4) and the second pole (5).

**10.** The toilet rim block (1) according to at least one of the preceding claims, wherein the toilet rim block (1) in each case has a sphericity of between 0.8 and 1.

11. The toilet rim block (1) according to at least one of the preceding claims, wherein the first and the second composition differ by a different concentration of the same active ingredient, wherein the core (3) has a concentration of an active ingredient that is at least 0.5 wt.%, preferably at least 1 wt.% to 10 wt.%, higher.

12. A rim block cage comprising at least two, preferably at least three, toilet rim blocks (1) according to at least one of the preceding claims, wherein the toilet rim blocks (1), which each comprise at least one pole (4, 5), are arranged in a regular or irregular manner with respect to one another.

13. The rim block cage according to claim 12, wherein the toilet rim blocks (1) are arranged in an irregular manner relative to one another.

**Revendications**

1. Bloc W.C. (1) constitué d'au moins une coque (2) et un noyau (3),

   dans lequel la coque (2) comprend une première composition et le noyau (3) comprend une seconde composition,
   dans lequel les première et seconde compositions diffèrent de préférence par au moins l'une des caractéristiques suivantes ; principes actifs différents, concentration différente d'un même principe actif, viscosité différente ;
   dans lequel la coque (2) enveloppe partiellement le noyau (3),
   dans lequel le noyau (3) fait saillie jusqu'à la surface sur deux côtés du bloc W.C., respectivement sous la forme d'un pôle (4), et est ainsi exposé
   **caractérisé en ce que**
   le bloc W.C. est sphérique, et
   le second pôle (5) fait saillie jusqu'à la surface sur le côté du bloc W.C. (1) opposé au premier pôle (4) et est ainsi exposé, dans lequel la coque (2) enveloppe le noyau (3) circulairement, et dans lequel le parcours du noyau (3) est conçu de sorte que la surface de l'aire exposée change lorsque la surface du bloc W.C. (1) est retirée uniformément.

2. Bloc W.C. (1) selon la revendication 1, dans lequel la surface de l'aire exposée du noyau (3) augmente lorsque la surface du bloc W.C. (1) est retirée uniformément.

3. Bloc W.C. (1) selon la revendication 1, dans lequel la surface de l'aire exposée du noyau (3) diminue lorsque la surface du bloc W.C. (1) est retirée uniformément.

4. Bloc W.C. (1) selon la revendication 3, dans lequel le rapport d'aire de la surface exposée du noyau (3) à la surface exposée de la coque (2) dans le bloc W.C. (1) non dissous est compris entre 5 % et 67 %, plus préférablement entre 10 % et 38 %.

5. Bloc W.C. (1) selon la revendication 3 et/ou 4, dans lequel le rapport d'aire de la surface exposée du noyau (3) à la surface exposée de la coque (2) dans le bloc W.C. (1) partiellement utilisé est compris entre 1:3 et 1:1.

6. Bloc W.C. (1) selon au moins l'une des revendications précédentes, dans lequel au moins une section, de préférence la section centrale du noyau (3), est réalisée sous la forme d'un segment cylindrique (6).

7. Bloc W.C. (1) selon au moins l'une des revendications précédentes, dans lequel le premier pôle (4) et le second pôle (5) sont reliés par un noyau (3) en forme de cordon.

8. Bloc W.C. (1) selon au moins l'une des revendications précédentes, dans lequel le parcours de section transversale du noyau (3) présente une réduction de section transversale entre le premier pôle (4) et le second pôle (5).

9. Bloc W.C. (1) selon au moins l'une des revendications 1 à 7, dans lequel le parcours de section transversale du noyau (3) présente un élargissement de section transversale entre le premier pôle (4) et le second pôle (5).

10. Bloc W.C. (1) selon au moins l'une des revendications précédentes, dans lequel le bloc W.C. (1) présente respectivement une sphéricité entre 0,8 et 1.

**11.** Bloc W.C. (1) selon au moins l'une des revendications précédentes, dans lequel la première et la seconde composition diffèrent par une concentration différente d'un même principe actif, dans lequel le noyau (3) présente une concentration plus élevée d'un principe actif d'au moins 0,5 % en poids, de préférence d'au moins 1 % en poids à 10 % en poids.

**12.** Cuvette de W.C. comportant au moins deux, de préférence au moins trois, blocs W.C. (1) selon au moins l'une des revendications précédentes, dans laquelle les blocs W.C. (1) sont disposés régulièrement ou irrégulièrement les uns par rapport aux autres avec respectivement au moins un pôle (4, 5).

**13.** Cuvette de W.C. selon la revendication 12, dans laquelle les blocs W.C. (1) sont disposés irrégulièrement les uns par rapport aux autres.

Fig. 1

Fig. 2

A-A

Fig. 3

B-B

Fig. 4

Fig. 5

Fig. 6

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0791047 B1
- DE 102008028138 A1
- WO 2010018006 A
- WO 2006070209 A
- WO 2012062914 A
- US 6376442 B

**In der Beschreibung aufgeführte Nicht-Patentliteratur**

- International Cosmetic Ingredient Dictionary and Handbook. The Cosmetic, Toiletry, and Fragrance Association (CTFA), 1997